# EUROPEAN PATENT SPECIFICATION

(11) **EP 2 446 905 B1**
(45) Date of publication and mention of the grant of the patent: **02.10.2019**
(21) Application number: 10189453.3
(22) Date of filing: 29.10.2010
(51) Int. Cl.: A61L 17/00, A61L 17/14

(54) **Medical device having anti-scarring properties**
Medizinische Vorrichtung mit Antikratzmerkmalen
Dispositif médical doté de propriétés anti-cicatrice

(43) Date of publication of application: 02.05.2012
(73) Proprietor: Aesculap AG, 78532 Tuttlingen/Donau (DE)
(72) Inventor: Odermatt, Erich, 8200 Schaffhausen (CH); Funk, Lutz, 08172 Sant Cugat del Vallés (ES); Sanchez, Ricardo, 08330 Premia de Mar (ES)
(74) Representative: Patentanwälte Ruff, Wilhelm, Beier, Dauster & Partner mbB

(56) References cited:
- EP-A1- 1 508 314
- EP-A1- 1 955 720
- EP-A2- 1 260 534
- WO-A1-2004/002549
- WO-A2-2007/089878
- WO-A2-2008/052179
- WO-A2-2010/088699
- JP-A- 2010 233 807
- US-A1- 2005 244 479
- US-A1- 2010 015 200

## Description

The present invention relates to a wound closure device for use in the medical improvement of scars and to a method for its manufacture.

Scarring is a commonly occurring problem and may cause severe medical problems. For instance, the appearance of scar tissue in the eye may result in hazy vision or blindness. In the peripheral and central nerval system, scarring may impair neuronal reconnections resulting in an impaired restoration of neuronal functions. In the reproductive organs scarring may cause infertility. In ligaments and tendons scarring can impede mechanical function and restrict the mobility of a patient.

In case of a dermal scarring, an adverse medical outcome may have tremendous psychological effects upon patients.

Therefore, many therapeutic approaches have been developed during the last decades in order to attenuate the extent of scar formation.

A cosmetic method for reducing or improving the appearance of cutaneous scar tissue requiring the application of lipoic acid is known from EP 1 039 891 B1.

The non-surgical approach disclosed in WO 2010/063994 A1 is based on the anti-scarring effects of TGF-β3 (Transforming Growth Factor Beta 3).

A topical scar treatment using a silicone composition is known from WO 01/23011 A1.

A scar treatment which relies on using blistering agents such as protein phosphatase inhibitors is known from WO 2007/149543 A2.

WO 98/39020 A1 discloses methods for treating and reducing the appearance of scar tissue with streptolysin O.

Apart from the above-referenced non-surgical scar revision techniques, surgical revision techniques such as surgical exision, dermabrasion, laser treatment and cryotherapy have also been developed.

However, surgical revision techniques often encounter the problem that patients who have already been subject to the trauma of scarring are frequently highly averse to undergoing additional surgical procedures that will, necessarily, result in further scarring.

WO 2008/052179 A2 discloses medical devices with polymer coatings designed to control the release of bioactive agents in combination with angiotensin-(1-7) receptor agonists.

WO 2004/002549 A1 relates to drug delivery systems comprising an angiotensin II antagonist (ARB) or a renin inhibitor (RI), or at least two representatives selected from the group consisting of an ARB, an angiotensin converting enzyme inhibitor (ACE-inhibitor) and a RI for the prevention and treatment of proliferative diseases.

US 2005/0244479 A1 pertains to biocompatible intraocular implants including an alpha-2 adrenergic receptor agonist and a polymer associated with the alpha-2 adrenergic receptor agonist to facilitate release of the alpha-2 adrenergic receptor agonist into an eye for an extended period of time.

JP 2010 233807 A discloses a stent having a physiologically active substance, wherein the substance may be an ACE inhibitor.

EP 1 508 314 A1 discloses an intravascular treatment device comprising at least one therapeutic agent, wherein the therapeutic agent may be an ACE inhibitor such as captopril.

EP 1 260 534 A2 discloses wound closure devices comprising a drug delivery coating, wherein the drug can be an antihypertensive drug.
EP 1 955 720 A1 discloses a barbed suture comprising a coating including a bioactive agent.

US 2010/015200 A1 refers to coated implantable medical devices, wherein the coating comprises at least one pharmaceutical agent, wherein the pharmaceutical agent may be an angiotensin converting enzyme inhibitor such as captopril.

In view of the foregoing, it is the object of the present invention to provide a medical device which contributes to wound healing accompanied by a minimized appearance of scar formation.

This object is achieved by a wound closure device with the features of independent claim 1 and a method for the manufacture of the wound closure device with the features of independent claim 13. Advantageous embodiments of the invention result from dependent claims 2 to 12. The wording of all claims is incorporated in this description by reference.

In terms of the device, the object is achieved by a wound closure device, comprising a device body, in particular an elongate device body, and a monomeric antihypertensive agent.

The term "wound closure device", as used according to the invention, encompasses any kind of device which enables the closure of wounds which may be classified as internal and/or external, in particular dermal, wounds.

The term "antihypertensive agent", as used according to the invention, may not only encompass the agent in its neutral form but also salts thereof.

The term "monomeric antihypertensive agent", as used according to the invention, is to be understood as an antihypertensive agent that is present as a monomer and not as an oligomer (such as a dimer, trimer, te-tramer, and the like) or as a polymer.

Further, the term "monomeric antihypertensive agent", in the following often abbreviated as "antihypertensive agent", as used according to the invention, may not only comprise one type of monomeric antihypertensive agent but also several types of monomeric antihypertensvive agents, i.e. a mixture of different antihypertensive agents. Differences may be derived from different agent classes, chemical structures, and the like.

The present invention is based on the concept that non-oligomerized or non-polymerized antihypertensive agents may serve as anti-scarring or scar reducing agents. The invention is further based on the concept that this sort of antihypertensive agents may be used to confer anti-scarring or scar reducing properties upon medical devices in order to reduce the outcome of scarring which results from surgical techniques requiring the employment of said medical devices. By doing so, sophisticated oligomerization or polymerization processes for manufacturing anti-scarring agents may be avoided. Further, due to their monomeric nature, the antihypertensive agents according to the present invention exhibit a better short-time release which is especially beneficial regarding wound healing which is typically completed within 2 to 3 weeks. Thus, the anti-scarring effect of the monomeric antihypertensive agents may positively influence the outcome of scarring already during the process of wound healing.

Furthermore, due to the monomeric nature of the anihypertensive agents, a scar-free wound healing or a wound healing being accompanied by a minimized scar formation is supported only within a defined period of time and in particular is limited to a local area of need.

In contrast, oligomerized or polymerized antihypertensive agents require a considerably longer degradation time accompanied by a significantly slower release rate of the monomers bearing the risk that an anti-scarring effect would arrive not until the wound healing is completed and that the monomeric antihypertensive agents - due to their retarded release starting from the oligomer and polymer, respectively - reach undesired areas in the body of a patient.

Without wishing to be bound to any theory, it is supposed that the monomeric antihypertensive agents of the present invention reduce collagen disposition, fibrosis and attenuate scar tissue metabolic activity, thereby leading to a minimized outcome of scarring.

In general, the antihypertensive agent according to the invention is a low molecular weight compound. More specifically, the antihypertensive agent may have a molecular weight below 1000 g /mol, in particular below 800 g/mol, preferably below 600 g/mol, and more preferably below 450 g/mol.

In a preferred embodiment, the antihypertensive agent is not covalently incorporated into the chemical structure of a material, in particular a basic material or starting material, of which the wound closure device, in particular the device body and/or a coating of the device body, is made.

The antihypertensive agent may be enveloped, wrapped or otherwise surrounded by a material, in particular a basic material or starting material, of which the wound closure device, in particular the device body and/or a coating of the device body, is made.

In a further embodiment, the antihypertensive agent is attached covalently or non-covalently, for example by means of adhesion forces, to the surface, preferably external surface, of the wound closure device, particularly of the device body and/or of a coating of the device body. In order to allow for a superficial attachment of the antihypertensive agent to the surface of the wound closure device, the device may be subjected to a suitable surface treatment such as a plasma treatment.

The antihypertensive agent is selected from the group consisting of captopril, zofenopril, lisinopril, benazepril, cilazapril, moexipril, perindopril, quinapril, ramipril, spirapril, trandolapril, alacepril, desacetyl-alacepril, delapril, imidapril, rentiapril, temocapril, ceronapril, enalapril, moveltipril, pivalopril, despivaloyl-pivalopril, fosinopril, salts thereof and mixtures thereof.

More preferably, the antihypertensive agent is selected from the group consisting of captopril ((*2S*)-1-[(*2S*)-2-methyl-3-sulfanylpropanoyl] pyrrolidine-2-carboxylic acid), ramipril ((2*S*,3a*S*,6a*S*)-1-[(2*S*)-2-{[(2*S*)-1-ethoxy-1-oxo-4-phenylbutan-2-yl]amino}propanoyl]-octahydrocyclo-penta[*b*]pyrrole-2-carboxylic acid), enalapril ((2*S*)-1-[(2*S*)-2-{[(2*S*)-1-ethoxy-1-oxo-4-phenylbutan-2-yl]amino}propanoyl]pyrrolidine-2-carboxylic acid), salts thereof and mixtures thereof.

In an especially preferred embodiment, the antihypertensive agent is captopril or a salt thereof.

Generally, the antihypertensive agent may be distributed, in particular homogeneously distributed, within the wound closure device, particularly within the device body.

Preferably, the antihypertensive agent is located or arranged onto the wound closure device, in particular onto the device body. More specifically, the antihypertensive agent may be located onto an external surface, i.e. a surface being visible from the outside, of the wound closure device, in particular of the device body. This advantageously allows for an immediate release of the antihypertensive agent into the environment of the wound closure device after being placed into the body of a patient.

As an alternative or in combination, the antihypertensive agent may be arranged onto an internal surface of the wound closure device, in particular of the device body. The term "internal surface" according to the invention is preferably understood as a surface surrounding a hollow space such as a passageway, channel, tunnel and/or pores of the wound closure device, in particular of the device body.

Further, the wound closure device comprises a biodegradable coating. The antihypertensive agent is included in the coating. The coating coats at least partly, particularly completely, the device body, in particular an internal and/or external surface, more preferably an external surface, thereof.

In a preferred embodiment, the antihypertensive agent is merely present in the coating.

On the other hand, the device body is preferably free of the antihypertensive agent.

In a further embodiment, the coating has a multilayer structure. Preferably the antihypertensive agent is included in at least one layer of the multilayer structure. More preferably, the antihypertensive agent is included in one or more surface layers, i.e. layers that are close to the surface, typically external surface, of the wound closure device.

The coating is typically a non-textile coating. However, textile coatings are principally not excluded by the present invention. For example, the coating may be present as a layer or multilayer structure, film, foil, in particular self-adhesive foil, strip, in particular self-adhesive strip, tape, in particular self-adhesive tape, paste, cream, impregnation, and the like.

In a further embodiment, the coating may have a porous structure, wherein the antihypertensive agent is preferably present in the pores of the coating. More specifically, the antihypertensive agent may coat the walls of the pores. A porous coating may be, for example, produced by means of a salt leaching technique.

According to a more specific embodiment, the coating is a sheath. More specifically, the wound closure device may comprise a core-sheath structure, wherein the core is typically constituted by the device body. In general, the antihypertensive agent may be included in the core and/or in the sheath. Preferably, the antihypertensive agent is included in the sheath.

In an advantageous embodiment, the coating is designed as a coating which softens upon contact with body liquids such as blood and other wound liquids and/or which softens upon exposure to the body temperature of a patient.

The antihypertensive agent may be present in the coating in a proportion from 0.1 % by weight to 25 % by weight, in particular from 0.1 % by weight to 15 % by weight, preferably from 0.25 % by weight to 15 % by weight, in particular from 0.25 % by weight to 10 % by weight, more preferably from 0.5 % by weight to 10 % by weight, in particular from 0.5 % by weight to 5 % by weight, relating to the total weight of the coating.

In a further embodiment, the coating has a proportion from 0.4 % by weight to 20 % by weight, in particular from 1 % by weight to 7 % by weight, preferably from 2 % by weight to 5 % by weight, relating to the total weight of the wound closure device.

The coating is preferably made of a biodegradable material. Typically, the coating is made of a polymer or copolymer. The term "polymer", as used according to the invention, may encompass synthetic polymers as well as biopolymers, i.e. natural polymers. The term "copolymer", as used according to the invention, defines a polymer which is composed of at least two different monomer units. Thus, for example, tripolymers, tetrapolymers, and the like are also encompassed by the term "copolymer". More specifically, the polymer can be present as a block or segmented copolymer, a random copolymer or a graft copolymer. Moreover, the polymer may be present as an isotactic, a syndiotactic or an atactic copolymer.

Advantageously, the coating is made of a polymer having a lower melting point than a polymer of which the device body is made. Thus, for example, the wound closure device according to the invention may be manufactured by extruding a coating including the antihypertensive agent at lower temperatures than the polymer which is used for the device body. By doing so, the antihypertensive agent is advantageously exposed to less harsh conditions during the manufacture of the wound closure device. Thus, the risk may be minimized that the functionality, in particular the anti-scarring or scar reducing properties, of the antihypertensive agent is impaired during the manufacturing process of the wound closure device.

More preferably, the coating is made of a biodegradable polymer. The term "biodegradable", as used according to the invention, preferably relates to the at least partial degradation or decomposition, i.e. chemical breakdown, of a polymer when being exposed to in vivo influences prevailing within a patient's body.

In case that the coating includes the antihypertensive agent, it is especially preferred that the coating is made of a fast biodegradable polymer so as to facilitate a fast release of the antihypertensive agent, and thereby an accelerated anti-scarring effect already during the initial wound healing phase. The term "fast biodegradable", as used according to the invention, defines an in vivo degradation time, i.e. a degradation time within a patient's body, within 1 day and 90 days, in particular 1 day and 60 days, and preferably 1 day and 21 days. In some cases, it may also be beneficial to achieve a long-term release of the antihypertensive agents. In such cases, the coating may be made of a slow biodegradable polymer. Thus, depending on the coating polymer, the release rate of the antihypertensive agent may be advantageously controlled. The optimal release period of the antihypertensive agent is preferably between 7 days and 14 days.

In a further embodiment, the coating is made of a biodegradable polymer selected from the group consisting of polyester such as polyhydroxyalkanoate, polydioxanone, polytrimethylene carbonate, polycarbonate, polyaminoacid, polyorthoester, polyethylene glycol, polyethylene oxide, polyvinyl pyrrolidone, polyvinylalcohol, polyphosphazene, polyether, polyalkylene oxalate, polyurethane, polyesteramide, polyamide, polyorthocarbonate, polyphosphoester, polyacetal, polyketal, polysaccharide, in particular mucopolysaccharide, protein, in particular extracellular protein, salts thereof, copolymers thereof and mixtures, in particular blends, thereof.

A suitable polyhydroxyalkanoate may be selected from the group consisting of polyglycolide, polylactide, polycaprolactone, polyhydroxybutyrate, in particular poly-3-hydroxybutyrate and/or poly-4-hydroxybutyrate, polyhdroxyvalerate, polyhydroxyhexanoate, salts thereof, copolymers thereof and mixtures, in particular blends, thereof.

A suitable polysaccharide may be selected from the group consisting of dextran, amylose, amylose pectin, starch, chitosan, methyl cellulose, carboxymethyl cellulose, hydroxymethyl cellulose, hydroxyethyl cellulose, hydroxyethyl methyl cellulose, hydroxypropyl cellulose, hydroxypropyl methyl cellulose, hydroxybutyl cellulose, hyaluronic acid, heparin, heparin sulphate, chondroitin-4 sulphate, chondroitin-6 sulphate, derma-tan sulphate, keratan sulphate, salts thereof, and mixtures thereof.

A suitable protein may be selected from the group consisting of collagen, gelatin, elastin, reticulin, fibronectin, albumin, salts thereof, and mixtures thereof.

The coating is preferably made of polydioxanone. Polydioxanone as a coating material is preferred in terms of its relatively low melting temperature.

In an alternative preferred embodiment, the coating is made of a copolymer made of glycolide and lactide, in particular having a proportion ratio from 9 : 1 to 1 : 9, preferably 7 : 3 to 3 : 7. Said copolymer is especially preferred if a fast biodegrading coating is desired.

Polyhydroxybutyrates may be generally chosen as coating polymers if a slow biodegrading coating is desired.

Further, a coating consisting of or essentially consisting of the antihypertensive agent may also be envisaged by the present invention, in particular in the case that the coating is a cream or paste as already mentioned.

The device body may be made of a non-biodegradable, partially biodegradable or completely biodegradable material.

Preferably, the device body is made of a polymer or copolymer.

More specifically, the device body may be made of a non-biodegradable polymer which is selected from the group consisting of polyolefin, polyester, polyamide, polyurethane, acrylate, methacrylate, pyrrolidone, polyoxyethylene, salts thereof, copolymers thereof and mixtures, in particular blends, thereof.

A suitable polyolefin may be selected from the group consisting of polyethylene, low-density polyethylene, high-density polyethylene, high-molecular-weight polyethylene, ultra-high-molecular-weight polyethylene, polypropylene, polytetrafluoroethylene, polyvinylidene dichloride, polyvinylidene difluoride, polytetrafluoropropylene, polyhexafluoropro-pylene, copolymers thereof and mixtures, in particular blends, thereof.

A suitable polyester may be selected from the group consisting of polyethylene terephthalate, polypropylene terephthalate, polybutylene terephthalate, copolymers thereof and mixtures, in particular blends, thereof.

Alternatively, the device body is made of a biodegradable polymer selected from the group consisting of polyester such as polyhydroxyalkanoate, polydioxanone, polytrimethylene carbonate, polycarbonate, polyaminoacid, polyorthoester, polyethylene glycol, polyethylene oxide, polyvinyl pyrrolidone, polyvinylalcohol, polyphosphazene, polyether, polyalkylene oxalate, polyurethane, polyesteramide, polyamide, polyorthocarbonate, polyphosphoester, polyacetal, polyketal, polysaccharide, in particular mucopolysaccharide, protein, in particular extracellular protein, salts thereof, copolymers thereof and mixtures, in particular blends, thereof.

A suitable polyhydroxyalkanoate may be selected from the group consisting of polyglycolide, polylactide, polycaprolactone, polyhydroxybutyrate, in particular poly-3-hydroxybutyrate and/or poly-4-hydroxybutyrate, polyhdroxyvalerate, polyhydroxyhexanoate, salts thereof, copolymers thereof and mixtures, in particular blends, thereof.

A suitable polysaccharide may be selected from the group consisting of dextran, amylose, amylose pectin, starch, chitosan, methyl cellulose, carboxymethyl cellulose, hydroxymethyl cellulose, hydroxyethyl cellulose, hydroxyethyl methyl cellulose, hydroxypropyl cellulose, hydroxypropyl methyl cellulose, hydroxybutyl cellulose, hyaluronic acid, heparin, heparin sulphate, chondroitin-4 sulphate, chondroitin-6 sulphate, dermatan sulphate, keratan sulphate, salts thereof, and mixtures thereof.

A suitable protein may be selected from the group consisting of collagen, gelatin, elastin, reticulin, fibronectin, albumin, salts thereof, and mixtures thereof.

In an especially preferred embodiment, the device body is made of polydioxanone or a terpolymer, in particular block terpolymer, made of glycolide, trimethylene carbonate and ε-caprolactone (such a terpolymer is, for example, commercially available under the trademark Monosyn®).

In a further embodiment, the device body is made of a metal such as titanium or of an alloy such as steel, particularly stainless steel.

It may be further within the scope of the present invention that the device body is made of a mixture including a non-biodegradable and a biodegradable material, in particular as described in the preceding embodiments.

In general, the device body and the coating may be made of different materials, in particular polymers, or of the same material, in particular the same polymer. According to the invention, it may be preferred that the device body, preferably being present as a core, and the coating, preferably being present as a sheath, are made of the same polymer, for example are made each of polydioxanone.

In a further preferred embodiment, the wound closure device, in particular the device body and/or a coating as described before, comprises a plurality of anchoring structures. The anchoring structures preferably protrude from the wound closure device, in particular from the device body and/or a coating as described before, especially from an internal and/or external surface thereof. In this embodiment, the term "internal surface" preferably relates to an internal surface of a hollow space of the device body.

The term "anchoring structures", as used according to the invention, preferably relates to structures which confer knotless, self-fixating or self-anchoring properties upon the wound closure device. Due to a typically pointed head, the anchoring structures penetrate tissue, thereby facilitating self-anchorage of the implant. Thus, additional fixing or anchoring steps such as knotting, gluing, welding, and the like, are not absolutely necessary to allow for a secure anchorage of the wound closure device in a patient's body.

As already mentioned before, the anchoring structures are preferably arranged as protrusions. More specifically, the anchoring structures may be designed as barbs, thorns, in particular rose-like thorns, hooks, spikes, darts, escutcheons, shields, scales, wedges, darts, arrows or combinations thereof.

Further, the anchoring structures may be configured in different dispositions onto the wound closure device, in particular onto the device body and/or onto a coating as described before. Suitable dispositions may be selected from the group consisting of a row disposition, a staggered disposition, an overlapping disposition, a random disposition, a helical or spiral disposition, an offset disposition, and offset and partially overlapping disposition, a zigzag disposition, a meander-like disposition, and combinations thereof.

Furthermore, the anchoring structures may be disposed in a unidirectionally disposition or in a multidirectionally, in particular bidirectionally, disposition onto the wound closure device, in particular onto the device body and/or onto a coating as described before.

The anchoring structures may be formed using any suitable method, including injection molding, stamping, cutting, laser, and the like. Preferably, the anchoring structures are designed as cuts made into the wound closure device, in particular into the device body and/or into a coating as described before. Moreover, the anchoring structures may be produced while the wound closure device, in particular the device body, is present in an undrawn or drawn state.

More specifically, the anchoring structures may be coated by a coating including the antihypertensive agent, wherein the coated anchoring structures are still visible as protrusions.

In a further embodiment, the device body is a mass body.

Further, the device body may be a porous body.

In an alternative embodiment, the device body is a hollow body comprising a hollow space. In particular, the hollow body may be designed as a multilayer construct. Preferably, at least one layer of the construct includes the antihypertensive agent. More preferably, the antihypertensive agent is included in one or more surface layers, i.e. layers that are close to the surface, particularly internal and/or external surface, typically external surface, of the construct. Preferably, the device body is a tubular or hose-like body.

Furthermore, the hollow body may have an internal diameter ranging between 0.05 mm and 1.1 mm, in particular 0.1 mm and 0.6 mm, preferably 0.2 mm and 0.5 mm. Moreover, the hollow body may have a wall thickness ranging between 0.02 mm and 0.9 mm, in particular between 0.05 mm and 0.6 mm, and preferably between 0.05 mm and 0.4 mm. The hollow space may beneficially serve as a reservoir for agents, preferably for the antihypertensive agent according to the present invention.

According to a more specific embodiment, the device body is a hollow body, wherein the internal surface of the hollow body is at least partly, preferably completely, lined with the antihypertensive agent or a coating including the antihypertensive agent.

In an alternative embodiment, the device body is a hollow body comprising a hollow space, wherein the hollow space comprises a filling. Preferably, the antihypertensive agent is included in the filling. It may be further within the scope of the present invention that the filling consists of the antihypertensive agent. For example, the filling may be a cream or a paste of the antihypertensive agent.

Instead of a filling as described in the preceding embodiment, the hollow space may comprise a physiological liquid, in particular solution or suspension, preferably including the antihypertensive agent.

Preferably, the hollow body comprises a plurality of anchoring structures, which are formed as through holes (breaking-throughs). In other words, the anchoring structures are preferably designed in such a way that they break through a wall of the hollow body. This embodiment is especially advantageous, by facilitating a release of the antihypertensive agent not only via open ends of the hollow body but also via said through holes. Thus, an increased release rate of the antihypertensive agent can be realised, advantageously during the initial phase of wound healing.

More specifically, the device body may be a wound closure device body, in particular a mechanical wound closure device body, such as a staple, wound strip, wound tape, wound foil, wound dressing or wound patch.

The device body is designed as a textile fabric, in particular selected from the group consisting of a non-woven fabric, a fleece, a felt, a knit fabric, in particular loop-formingly knit fabric, preferably a warp knit fabric, more preferably formed-loop warp-knit fabric, and a braided fabric.

The device body is a textile mesh.

The wound closure device is a surgical mesh such as a hernia mesh, prolaps mesh or incontinence sling.

In a further embodiment, the wound closure device, in particular the device body and/or a coating as described before, may additionally include further additives such as medical or pharmaceutical agents, antimicrobial, in particular antibiotic, agents, disinfecting agents, growth-promoting agents, anti-inflammatory agents, analgesic agents, odour-controlling agents, biologics such as proteins, enzymes, polysaccharides, polynucleotides, cells such as stem cells, and the like, or combinations thereof.

In an especially preferred embodiment, the wound closure device, in particular the device body and/or a coating as described before, includes fatty acids and/or salts thereof. Preferred fatty acid salts are alkaline metal salts of fatty acids and/or alkaline earth metal salts of fatty acids, in particular magnesium salts of fatty acids and/or calcium salts of fatty acids such as magnesium stearate and/or calcium stearate.

In a method for manufacturing or producing a wound closure device, according to which the object of the invention is achieved, an antihypertensive agent is added to a material, in particular a basic material or starting material, of a coating which is used to coat a device body.

Preferably, the coating including the antihypertensive agent is applied to the device body, in particular to an internal and/or external surface thereof.

Generally, different techniques may be used in order to apply the coating including the antihypertensive agent to the device body. Suitable application techniques may comprise coating, impregnation, immersion, soaking, wetting, dipping, spraying, brushing, rolling and/or calendaring. Depending on the respective application technique, it may be advantageous to use a liquid dispersion, suspension, solution or melting including the antihypertensive agent.

In a preferred embodiment, the wound closure device is manufactured by means of core-sheath extrusion. Preferably the core-sheath extrusion is conducted in such a way that the sheath includes the antihypertensive agent. The sheath may advantageously be extruded at lower temperatures than the core.

In an alternative embodiment, the wound closure device is manufactured by means of sheath extrusion, i.e. a sheath is extruded onto an already formed core, i.e. onto an already formed device body. Preferably, the sheath includes the antihypertensive agent.

In yet a further embodiment, a hollow space of the device body is filled with a filling including the antihypertensive agent or with a filling consisting of the antihypertensive agent. Preferably, the filling is present as a cream or paste.

With respect to further features and advantages of the wound closure device, in particular in view of the device body and/or the antihypertensive agent, reference is made in its entirety to the previous description.

With respect to further features and advantages of the wound closure device, in particular in view of the device body and/or the antihypertensive agent, reference is also made in its entirety to the previous description.

Further features and advantages of the invention may be found in the following description of preferred embodiments in the form of examples and figure descriptions. The corresponding figures are hereby made part of the content of this description by explicit reference. Individual features may be respectively implemented on their own, or several together in combination with one another. The examples and figures including the corresponding figure descriptions merely serve to explain the present invention without in any way restricting thereto.

The figures schematically depict:
- Fig. 1:: an embodiment of a medical device,
- Fig. 2A:: a further embodiment of a medical device ,
- Fig. 2B:: a further embodiment of a medical device,
- Fig. 3A:: a further embodiment of a medical device,
- Fig. 3B:: a further embodiment of a medical device, and
- Fig. 4:: an embodiment of the wound closure device according to the present invention.

### Examples

### Example 1: Tube with self fixating elements coated by an antihypertensive agent containing biodegradable coating for implantation (not belonging to the invention).

A tube made from the biodegradable polymer poly-p-dioxanone, having a diameter of the lumen of 450 µm and a wall thickness of 230 µm, is cut into strands of 15 cm length. As a next step cuts are made into the tube to provide the tube with anchoring structures that allow a "knotless fixation" of the tube in the tissue, with the cuts reaching into the lumen of the tube. The cuts are prepared in a way that one group of the structures is pointing to one direction along the tube and another group is pointing to the opposite direction (bi-directional anchoring structures). After the cutting process the tube is stretched 4-fold along the tubes longitudinal axis in a hot air oven at 70 ºC. Upon drawing the anchoring structures erect and the cuts open and give access to the lumen of the tube. The accordingly prepared tube is submerged for a short time in a stirred bath containing 5% w/w poly-4-hydroxybutyrate and 0,1% w/w captopril in chloroform at 35 ºC bath temperature. The tube structure and cuts in the wall of the tube allow a uniform wetting of the lumen as well as of the outer surface of the tube by the coating solution. After the dip coating process the coated tube is dried at 40 ºC in a hot air oven. If needed the dip coating and drying process can be repeated to obtain a thicker coating layer on the tube structure.

### Example 2: Coating application on sutures (not belonging to the invention).

A thread of polyglycolic braided absorbable suture (Safil) passes through a coating dispersion bath containing captopril 0.3% w/v and 6% w/v poly(lactide-co-glycolide) 70:30 in ethyl acetate at 25 ºC (the coating dispersion is under continuous stirring). After passing through the coating bath, the thread passes through a drying channel which has an interior temperature around 100 ºC where the solvent is evaporated (the drying channel is surrounded by a calefaction system connected to a thermic oil boiler). After passing through the drying channel, the dried thread containing the coating which includes the active agent is collected onto a bobbin.

### Example 3: Coating application on meshes

### Example 3.1: Coating of a Safil mesh

From the roll, the knitted mesh of polyglycolic acid (Safil mesh) passes through a tank containing the coating dispersion of Captopril 0.25% w/v and 4% w/v poly(lactide-co-glycolide) 70:30 in ethyl acetate at 25 ºC (the coating dispersion is under continuous stirring). After passing through the coating bath, the mesh fabric passes through two rolls (foulard system) that are in contact and rolling in the same direction and applying a certain pressure to the fabric mesh for eliminating the excess of absorbed coating and assure a homogenized coated surface. Afterwards the mesh fabric is driven into an IR irradiation oven which has an interior temperature around 100 ºC where the solvent is evaporated. Finally the mesh fabric containing the coating which includes the active agent is collected onto a roll.

### Example 3.2: Coating of an Optilene LP mesh

From the roll, the knitted mesh of polypropylene (Optilene LP) passes through a tank containing the coating dispersion of captopril 0.25% w/v in isopropanol at 40 °C. The solution is under continuous stirring. After passing through the coating bath, the mesh fabric passes through two rolls (foulard system) that are in contact and rolling in the same direction and applying a certain pressure to the fabric mesh for eliminating the excess of absorbed coating and assure a homogenized coated surface. Afterwards the mesh fabric is driven into a IR irradiation oven which has an interior temperature around 60 ºC where the solvent is evaporated. Finally the mesh fabric containing the coating which includes the active agent is collected onto a roll and visually controlled.

### Description of the figures

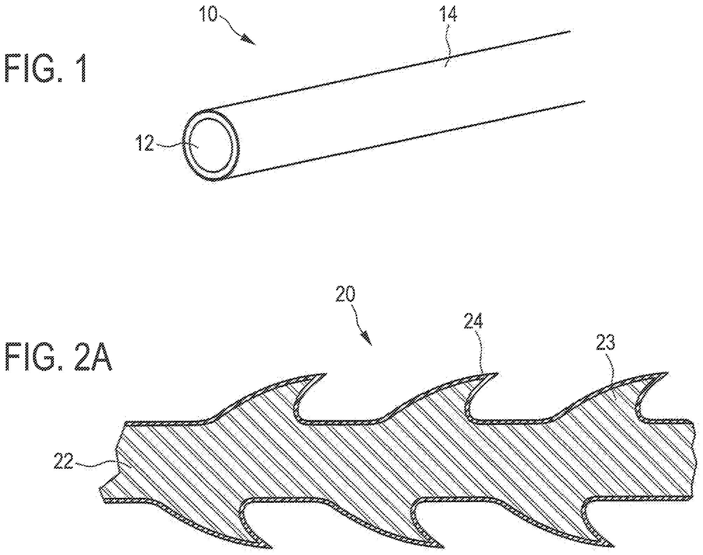

Figure 1 schematically depicts a medical device 10 in the form of a surgical thread comprising a thread body 12 and comprising a coating 14. The coating 14 is typically biodegradable. The coating 14 includes a monomeric antihypertensive agent, preferably the angiotensin-I converting enzyme inhibitor captopril, a salt thereof or a derivative thereof. The antihypertensive agent may be included in the coating 14 in a proportion ranging between 0.1 % by weight and 15 % by weight, in particular between 0.25 % by weight and 10 % by weight, and preferably between 0.5 % by weight and 5 % by weight.

Typically, the thread body 12 is completely coated by the coating 14.

In order to facilitate a fast release of the antihypertensive agent into a wound site, the coating 14 is preferably made of a fast biodegrading polymer such as a copolymer made of glycolide and lactide, in particular having a proportion ratio from 9 : 1 to 1 : 9, preferably 7 : 3 to 3 : 7.

Alternatively, it may be preferred that the coating 14 is made of polydioxanone.

The thread body 12 may be made of a non-biodegradable or biodegradable polymer depending on the surgical application. Preferably, the thread body 12 is made of a terpolymer, in particular block terpolymer, made of glycolide, trimethylene carbonate and ε-caprolactone. Such a terpolymer is, for example, commercially available under the trademark Monosyn®.

Alternatively, it may be preferred that the thread body 12 is made of polydioxanone.

In the case that a certain degree of stiffness of the surgical thread 10 is desired, the thread body 12 is preferably designed as a monofilament. A monofile thread body 12 bears the additional advantage that the risk of post-surgical infections may be minimized due to the poor capillarity of a monofilament.

If the surgical thread 10 should be more flexible, the thread body 12 is preferably designed as a multifilament, in particular as a braided multifilament or as a ply yarn. Further, in the case that a multifile thread body 12 is completely, i.e. all over, coated, the resulting surgical thread 10 is present as a pseudo monofilament, thereby additionally showing a poor capillarity which - as mentioned above - helps to avoid the occurrence of post-surgical infections.

The surgical thread 10 as shown in figure 1 may be the result of a core-sheath extrusion or merely of a sheath extrusion. If the surgical thread 10 is manufactured by means of core-sheath extrusion, the core is preferably made of a terpolymer made of glycolide, trimethylene carbonate and ε-caprolactone, whereas the sheath is preferably made of polydioxanone. Thus, polydioxanone including the antihypertensive agent may be extruded at lower temperatures than the afore-mentioned terpolymer implicating less harsh conditions for the antihypertensive agent.

With respect to further features and advantages of the surgical thread 10, reference is made in its entirety to the previous description.

Figure 2A schematically depicts a further embodiment of a medical device 20 in the form of a surgical thread comprising a thread body 22 having a plurality of anchoring structures 23 and comprising a coating 24. The coating 24 is typically biodegradable. The coating 24 includes an antihypertensive agent such as captopril, a salt thereof or a derivative thereof.

The anchoring structures 23 allow for a self-anchoring of the surgical thread 20. The self-anchorage is mainly based on the tissue penetrating effect of the anchoring structures 23. Thus, a knotting of the thread 20 or other anchoring techniques such as gluing, welding, and the like is not mandatory. The anchoring structures 23 may be designed as barbs, hooks, thorns, and the like. Generally, the anchoring structures 23 protrude from the thread body 22 (i.e. the external surface thereof). Moreover, the anchoring structures 23 are preferably designed as cuts into the thread body 22 and/or the coating 24.

Advantageously, the thread body 22 is coated in such a manner that the coated anchoring structures 23 are still visible as protrusions.

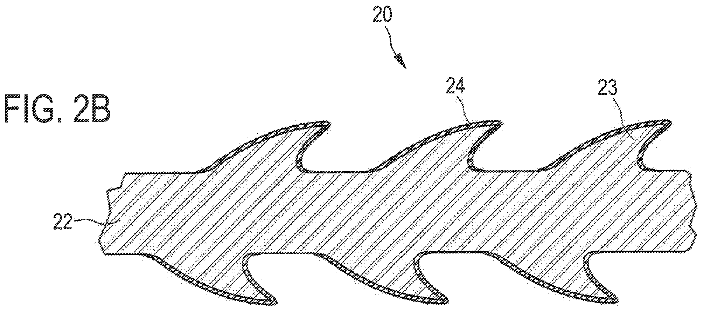

As depicted in figure 2A, the thread body 22 may be completely coated. However, it may also be within the scope of the invention that the thread body 22 is merely partially coated. For example, as schematically depicted in figure 2B, only the anchoring structures 23 may be coated. Thus, a localised release of the antihypertensive agent is possible allowing for the manifestation of an anti-scarring effect especially in the regions where the anchoring structures 23 penetrate and accordingly traumatize tissue.

With respect to further features and advantages of the surgical threads 20 depicted in the figures 2A und 2B, reference is made in its entirety to the previous description, in particular to the description of figure 1.

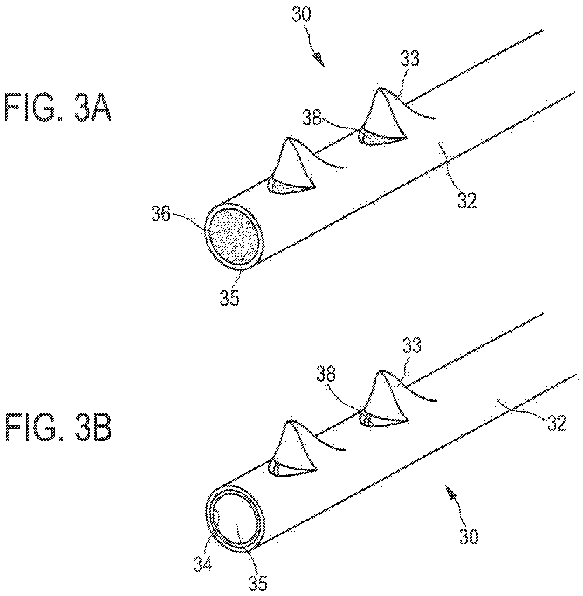

Figure 3A schematically shows a further embodiment of a medical device 30 in the form of a tubular medical device, in particular tubular wound closure device, comprising a tubular device body 32 and a filling 36 including or consisting of an antihypertensive agent such as captopril, a salt thereof or a derivative thereof. The tubular device body 32 comprises an external surface and an internal surface. Further, the tubular device body 32 comprises a hollow space 35 which is loaded or filled with the filling 36. The filling 36 is preferably present as a cream or paste. Further, the tubular device body 32 advantageously has an internal diameter ranging between 0.05 mm and 1.1 mm, in particular 0.1 mm and 0.6 mm, and preferably 0.2 mm and 0.5 mm. Furthermore, the tubular device body 32 may have a wall thickness from 0.02 mm to 0.9 mm, in particular from 0.05 mm to 0.6 mm, preferably, from 0.05 mm to 0.4 mm.

As an alternative to the filling 36 as described above, the hollow space 35 may be loaded with a solution or suspension including the antihypertensive agent.

The tubular device body 32 further comprises a plurality of anchoring structures 33, preferably being designed as barbs, hooks, thorns, and the like, which protrude from the external surface of the tubular device body 32. The anchoring structures 33 allow - as already described in the context of figures 2A and 2B - for a self-anchoring of the device 30. Moreover, the anchoring structures 33 are designed as through holes, i.e. are designed such that they completely break through the wall of the tubular device body 32, thereby forming holes 38 in the wall of the device body 32.

After placing the device 30 into the body of a patient, the antihypertensive agent may not only be released via the open ends of the tubular body 32 but also via the holes 38. This beneficially contributes to an enhanced release of the antihypertensive agent, and thus to an increased anti-scarring effect, in particular during the initial wound healing phase.

Figure 3B schematically depicts an alternative device 30, wherein a coating 34 including or consisting of the antihypertensive agent lines the internal surface of the device body 32. In the case that the coating 34 merely includes the antihypertensive agent, the coating 34 can be made of a suitable carrier material for the antihypertensive agent such as polysaccharides, in particular mucopolysaccharides, or proteins.

With respect to further features and advantages of the medical device 30, reference is made in its entirety to the previous description.

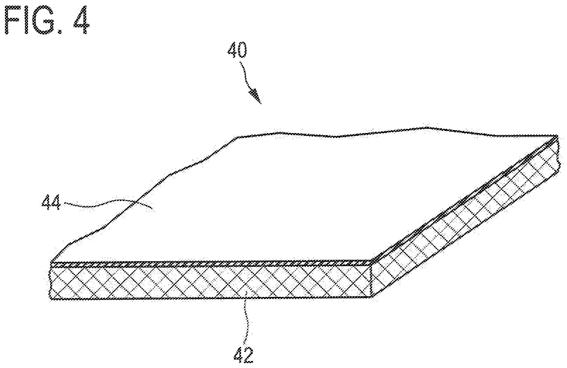

Figure 4 schematically depicts an embodiment of a medical device 40 according to the invention which is present as a two-dimensional surgical implant, in particular as hernia or prolapse implant, comprising a textile mesh 42 and comprising a coating 44 including captopril or a salt thereof. The mesh 42 is preferably made of polyester or polyolefin monofilaments such as polyethylene terephthalate monofilaments or polypropylene monofilaments. The coating 44 may be present only on one main surface of the surgical implant 40, i.e. the opposite main surface may be free of the coating 44.

For example, in the case that the surgical implant 40 is a hernia implant, it is preferred that only the main surface is coated which faces the abdominal cavity upon insertion of the implant 40 into the patient's abdomen. Thus, the risk that post-surgical adhesions may occur between the intestines and the implant 40 can be lowered, at the same time reducing the weight of the implant 40 by not coating both main surfaces thereof. Due to the anti-scarring effect of the antihypertensive agent included in the coating 44, the risk of post-surgical adhesions can be dramatically lowered. A suitable coating material is collagen, gelatine, polyurethane and/or polyvinylalcohol.

## Claims

1. Wound closure device comprising a device body and a monomeric antihypertensive agent, **characterized in that** the antihypertensive agent is included in a biodegradable coating of the device body and that the antihypertensive agent is selected from the group consisting of captopril, zofenopril, lisinopril, benazepril, cilazapril, moexipril, perindopril, quinapril, ramipril, spirapril, trandolapril, alacepril, desacetyl-alacepril, delapril, imidapril, rentiapril, temocapril, ceronapril, enalapril, moveltipril, pivalopril, despivaloyl-pivalopril, fosinopril, salts thereof and mixtures thereof and that the device body is designed as a textile mesh and the device is a surgical mesh.

2. Wound closure device according to claim 1, **characterized in that** the antihypertensive agent is not covalently incorporated into the chemical structure of a material, in particular a basic material or starting material, of which the coating is made.

3. Wound closure device according to claim 1 or 2, **characterized in that** the antihypertensive agent is an angiotensin-I converting enzyme inhibitor selected from the group consisting of captopril, ramipril, enalapril, salts thereof and mixtures thereof.

4. Wound closure device according to one of the preceding claims, **characterized in that** the antihypertensive agent is present in the coating in a proportion from 0.1 to 25 % by weight, in particular from 0.25 to 15 % by weight, preferably from 0.5 to 10 % by weight, relating to the total weight of the coating.

5. Wound closure device according toone of the preceding claims, **characterized in that** the coating is made of a biodegradable polymer, preferably selected from the group consisting of polyester such as polyhydroxyalkanoate, polydioxanone, polytrimethylene carbonate, polycarbonate, polyaminoacid, polyorthoester, polyethylene glycol, polyethylene oxide, polyvinyl pyrrolidone, polyvinylalcohol, polyphosphazene, polyether, polyalkylene oxalate, polyurethane, polyesteramide, polyamide, polyorthocarbonate, polyphosphoester, polyacetal, polyketal, polysaccharide, in particular mucopolysaccharide, protein, in particular extracellular protein, salts thereof, copolymers thereof and mixtures, in particular blends, thereof.

6. Wound closure device according to one of the preceding claims, **characterized in that** the coating is made of polydioxanone or of a copolymer made of glycolide and lactide, in particular having a proportion ratio from 9 : 1 to 1 : 9, preferably 7 : 3 to 3 : 7.

7. Wound closure device according to one of the preceding claims, **characterized in that** the device body is made of a biodegradable material, in particular biodegradable polymer, preferably selected from the group consisting of polyester such as polyhydroxyalkanoate, polydioxanone, polytrimethylene carbonate, polycarbonate, polyaminoacid, polyorthoester, polyethylene glycol, polyethylene oxide, polyvinyl pyrrolidone, polyvinylalcohol, polyphosphazene, polyether, polyalkylene oxalate, polyurethane, polyesteramide, polyamide, polyorthocarbonate, polyphosphoester, polyacetal, polyketal, polysaccharide, in particular mucopolysaccharide, protein, in particular extracellular protein, salts thereof, copolymers thereof and mixtures, in particular blends, thereof.

8. Wound closure device according to one of the preceding claims, **characterized in that** the device body is made of polydioxanone or a terpolymer made of glycolide, trimethylene carbonate and ε-caprolactone.

9. Wound closure device according to one of claims 1 to6, **characterized in that** the device body is made of a non-biodegradable material, in particular non-biodegradable polymer, preferably selected from the group consisting of polyolefin, polyester, polyamide, polyurethane, acrylate, methacrylate, pyrrolidone, polyoxyethylene, salts thereof, copolymers thereof, metals, alloys, and mixtures, in particular blends, thereof.

10. Wound closure device according to one of the preceding claims, **characterized in that** the device body comprises a plurality of anchoring structures, preferably barbs, thorns, hooks, and the like, which preferably protrude from the device body.

11. Wound closure device according to one of the preceding claims, **characterized in that** the device body is a hollow body, in particular a tube or hose, comprising a hollow space, wherein the hollow space is preferably loaded with a filling, particularly in the form of a cream or paste, including the antihypertensive agent.

12. Wound closure device according to one of the preceding claims, **characterized in that** the surgical mesh is a hernia mesh, prolaps mesh or incontinence sling.

13. Method for the manufacture of a wound closure device according to one of the preceding claims, **characterized in that** a monomeric antihypertensive agent is added to a material, in particular a basic material or starting material, of a coating which is used to coat a device body.

## Patentansprüche

1. Wundverschlussvorrichtung, umfassend einen Vorrichtungskörper und ein monomeres antihypertensives Mittel, **dadurch gekennzeichnet, dass** das antihypertensive Mittel in einer biologisch abbaubaren Beschichtung des Vorrichtungskörpers vorgesehen ist, und dass das antihypertensive Mittel ausgewählt ist aus der Gruppe bestehend aus Captopril, Zofenopril, Lisinopril, Benazepril, Cilazapril, Moexipril, Perindopril, Quinapril, Ramipril, Spirapril, Trandolapril, Alacepril, Desacetylalacepril, Delapril, Imidapril, Rentiapril, Temocapril, Ceronapril, Enalapril, Moveltipril, Pivalopril, Despivaloylpivalopril, Fosinopril, Salzen davon und Mischungen davon, und dass der Vorrichtungskörper als textiles Netz ausgebildet ist und es sich bei der Vorrichtung um ein chirurgisches Netz handelt.

2. Wundverschlussvorrichtung nach Anspruch 1, **dadurch gekennzeichnet, dass** das antihypertensive Mittel nicht kovalent in die chemische Struktur eines Materials, insbesondere eines Basismaterials oder Ausgangsmaterials, aus dem die Beschichtung gebildet ist, eingebracht ist.

3. Wundverschlussvorrichtung nach Anspruch 1 oder 2, **dadurch gekennzeichnet, dass** es sich bei dem antihypertensiven Mittel um einen Inhibitor des Angiotensin-I-konvertierenden Enzyms handelt, ausgewählt aus der Gruppe bestehend aus Captopril, Ramipril, Enalapril, Salzen davon und Mischungen davon.

4. Wundverschlussvorrichtung nach einem der vorhergehenden Ansprüche, **dadurch gekennzeichnet, dass** das antihypertensive Mittel in der Beschichtung in einem Anteil von 0,1 bis 25 Gewichts-%, insbesondere von 0,25 bis 15 Gewichts-%, bevorzugt von 0,5 bis 10 Gewichts-%, bezogen auf das Gesamtgewicht der Beschichtung vorliegt.

5. Wundverschlussvorrichtung nach einem der vorhergehenden Ansprüche, **dadurch gekennzeichnet, dass** die Beschichtung aus einem biologisch abbaubaren Polymer gebildet ist, bevorzugt ausgewählt aus der Gruppe bestehend aus Polyester wie Polyhydroxyalkanoat, Polydioxanon, Polytrimethylencarbonat, Polycarbonat, Polyaminosäure, Polyorthoester, Polyethylenglycol, Polyethylenoxid, Polyvinylpyrrolidon, Polyvinylalkohol, Polyphosphazen, Polyether, Polyalkylenoxalat, Polyurethan, Polyesteramid, Polyamid, Polyorthocarbonat, Polyphosphoester, Polyacetal, Polyketal, Polysaccharid, insbesondere Mucopolysaccharid, Protein, insbesondere extrazelluläres Protein, Salzen davon, Copolymeren davon und Mischungen, insbesondere Blends, davon.

6. Wundverschlussvorrichtung nach einem der vorhergehenden Ansprüche, **dadurch gekennzeichnet, dass** die Beschichtung aus Polydioxanon oder einem Copolymer gebildet aus Glycolid und Lactid, insbesondere in einem Anteilsverhältnis von 9 : 1 bis 1 : 9, vorzugsweise 7 : 3 bis 3 : 7, gebildet ist.

7. Wundverschlussvorrichtung nach einem der vorhergehenden Ansprüche, **dadurch gekennzeichnet, dass** der Vorrichtungskörper aus einem biologisch abbaubaren Material, insbesondere einem biologisch abbaubaren Polymer, gebildet ist, bevorzugt ausgewählt aus der Gruppe bestehend aus Polyester wie Polyhydroxyalkanoat, Polydioxanon, Polytrimethylencarbonat, Polycarbonat, Polyaminosäure, Polyorthoester, Polyethylenglycol, Polyethylenoxid, Polyvinylpyrrolidon, Polyvinylalkohol, Polyphosphazen, Polyether, Polyalkylenoxalat, Polyurethan, Polyesteramid, Polyamid, Polyorthocarbonat, Polyphosphoester, Polyacetal, Polyketal, Polysaccharid, insbesondere Mucopolysaccharid, Protein, insbesondere extrazelluläres Protein, Salzen davon, Copolymeren davon und Mischungen, insbesondere Blends, davon.

8. Wundverschlussvorrichtung nach einem der vorhergehenden Ansprüche, **dadurch gekennzeichnet, dass** der Vorrichtungskörper aus Polydioxanon oder einem Terpolymer gebildet aus Glycolid, Trimethylencarbonat und ε-Caprolacton gebildet ist.

9. Wundverschlussvorrichtung nach einem der Ansprüche 1 bis 6, **dadurch gekennzeichnet, dass** der Vorrichtungskörper aus einem nicht biologisch abbaubaren Material, insbesondere einem nicht biologisch abbaubaren Polymer, gebildet ist, bevorzugt ausgewählt aus der Gruppe bestehend aus Polyolefin, Polyester, Polyamid, Polyurethan, Acrylat, Methacrylat, Pyrrolidon, Polyoxyethylen, Salzen davon, Copolymeren davon, Metallen, Legierungen und Mischungen, insbesondere Blends, davon.

10. Wundverschlussvorrichtung nach einem der vorhergehenden Ansprüche, **dadurch gekennzeichnet, dass** der Vorrichtungskörper eine Mehrzahl von Verankerungsstrukturen umfasst, bevorzugt Widerhaken, Stacheln, Haken und dergleichen, die vorzugsweise vom Vorrichtungskörper abstehen.

11. Wundverschlussvorrichtung nach einem der vorhergehenden Ansprüche, **dadurch gekennzeichnet, dass** der Vorrichtungskörper als Hohlkörper ausgebildet ist, insbesondere als Röhre oder Schlauch, mit einem Hohlraum, wobei der Hohlraum bevorzugt mit einer Füllung versehen ist, insbesondere in Form einer Creme oder Paste, die das antihypertensive Mittel enthält.

12. Wundverschlussvorrichtung nach einem der vorhergehenden Ansprüche, **dadurch gekennzeichnet, dass** es sich bei dem chirurgischen Netz um ein Herniennetz, ein Prolapsnetz oder eine Inkontinenzschlinge handelt.

13. Verfahren zur Herstellung einer Wundverschlussvorrichtung nach einem der vorhergehenden Ansprüche, **dadurch gekennzeichnet, dass** ein monomeres antihypertensives Mittel zu einem Material, insbesondere einem Basismaterial oder Ausgangsmaterial, einer Beschichtung, die zum Beschichten eines Vorrichtungskörpers verwendet wird, zugegeben wird.

## Revendications

1. Dispositif de fermeture de plaie comprenant un corps de dispositif et un agent antihypertenseur monomère, **caractérisé en ce que** l'agent antihypertenseur est inclus dans un revêtement biodégradable du corps de dispositif et que l'agent antihypertenseur est choisi dans le groupe constitué des captopril, zofénopril, lisinopril, bénazépril, cilazapril, moexipril, périndopril, quinapril, ramipril, spirapril, trandolapril, alacépril, désacétyl-alacépril, délapril, imidapril, rentiapril, témocapril, céronapril, énalapril, moveltipril, pivalopril, despivaloyl-pivalopril, fosinopril, des sels de ceux-ci et des mélanges de ceux-ci et que le corps de dispositif est conçu sous la forme d'un treillis textile et que le dispositif est un treillis chirurgical.

2. Dispositif de fermeture de plaie selon la revendication 1, **caractérisé en ce que** l'agent antihypertenseur n'est pas incorporé de façon covalente dans la structure chimique d'un matériau, en particulier un matériau de base ou un matériau de départ, à partir duquel le revêtement est formé.

3. Dispositif de fermeture de plaie selon la revendication 1 ou 2, **caractérisé en ce que** l'agent antihypertenseur est un inhibiteur d'enzyme de conversion d'angiotensine 1 choisi dans le groupe constitué des captopril, ramipril, énalapril, des sels de ceux-ci et des mélanges de ceux-ci.

4. Dispositif de fermeture de plaie selon l'une des revendications précédentes, **caractérisé en ce que** l'agent antihypertenseur est présent dans le revêtement dans une proportion de 0,1 à 25 % en poids, en particulier de 0,25 à 15 % en poids, de préférence de 0,5 à 10 % en poids, par rapport au poids total du revêtement.

5. Dispositif de fermeture de plaie selon l'une des revendications précédentes, **caractérisé en ce que** le revêtement est constitué d'un polymère biodégradable, de préférence choisi dans le groupe constitué de polyester tel qu'un polyhydroxyalcanoate, polydioxanone, poly(carbonate de triméthylène), polycarbonate, polyaminoacide, polyorthoester, polyéthylène glycol, poly(oxyde d'éthylène), polyvinylpyrrolidone, alcool polyvinylique, polyphosphazène, polyéther, poly(oxalate d'alkylène), polyuréthane, polyesteramide, polyamide, polyorthocarbonate, polyphosphoester, polyacétal, polycétal, polysaccharide, en particulier un mucopolysaccharide, protéine, en particulier une protéine extracellulaire, des sels de ceux-ci, des copolymères de ceux-ci et des mélanges, en particulier des compositions, de ceux-ci.

6. Dispositif de fermeture de plaie selon l'une des revendications précédentes, **caractérisé en ce que** le revêtement est constitué de polydioxanone ou d'un copolymère constitué de glycolide et de lactide, en particulier ayant un rapport de proportion de 9:1 à 1:9, de préférence de 7:3 à 3:7.

7. Dispositif de fermeture de plaie selon l'une des revendications précédentes, **caractérisé en ce que** le corps de dispositif est constitué d'un matériau biodégradable, en particulier un polymère biodégradable, de préférence choisi dans le groupe constitué de polyester tel qu'un polyhydroxyalcanoate, polydioxanone, poly(carbonate de triméthylène), polycarbonate, polyaminoacide, polyorthoester, polyéthylène glycol, poly(oxyde d'éthylène), polyvinylpyrrolidone, alcool polyvinylique, polyphosphazène, polyéther, poly(oxalate d'alkylène), polyuréthane, polyesteramide, polyamide, polyorthocarbonate, polyphosphoester, polyacétal, polycétal, polysaccharide, en particulier un mucopolysaccharide, protéine, en particulier une protéine extracellulaire, des sels de ceux-ci, des copolymères de ceux-ci et des mélanges, en particulier des compositions, de ceux-ci.

8. Dispositif de fermeture de plaie selon l'une des revendications précédentes, **caractérisé en ce que** le corps de dispositif est constitué de polydioxanone ou d'un terpolymère constitué de glycolide, tri(carbonate de méthylène) et ε-caprolactone.

9. Dispositif de fermeture de plaie selon l'une des revendications 1 à 6, **caractérisé en ce que** le corps de dispositif est constitué d'un matériau non biodégradable, en particulier un polymère non biodégradable, de préférence choisi dans le groupe constitué de polyoléfine, polyester, polyamide, polyuréthane, acrylate, méthacrylate, pyrrolidone, polyoxyéthylène, des sels de ceux-ci, des copolymères de ceux-ci, des métaux, des alliages, et des mélanges, en particulier des compositions, de ceux-ci.

10. Dispositif de fermeture de plaie selon l'une des revendications précédentes, **caractérisé en ce que** le corps de dispositif comprend une pluralité de structures d'ancrage, de préférence des barbes, des épines, des crochets, et similaire, qui, de préférence, font saillie depuis le corps de dispositif.

11. Dispositif de fermeture de plaie selon l'une des revendications précédentes, **caractérisé en ce que** le corps de dispositif est un corps creux, en particulier un tube ou un tuyau flexible, comprenant un espace creux, dans lequel l'espace creux est, de préférence, chargé avec un remplissage, en particulier sous la forme d'une crème ou d'une pâte, comprenant l'agent antihypertenseur.

12. Dispositif de fermeture de plaie selon l'une des revendications précédentes, **caractérisé en ce que** le treillis chirurgical est un treillis à hernie, un treillis à prolapsus ou un anneau pour l'incontinence.

13. Procédé de fabrication d'un dispositif de fermeture de plaie selon l'une des revendications précédentes, **caractérisé en ce qu'**un agent antihypertenseur monomère est ajouté à un matériau, en particulier un matériau de base ou un matériau de départ, d'un revêtement qui est utilisé pour revêtir un corps de dispositif.
